Europäisches Patentamt

(19) European Patent Office

Office européen des brevets

(11) Publication number: **0 130 074**
**B1**

(12) **EUROPEAN PATENT SPECIFICATION**

(45) Date of publication of patent specification: 06.02.91

(51) Int. Cl.⁵: **C 12 N 15/70,** C 12 N 15/90, C 12 N 15/75, C 12 P 21/00

(21) Application number: 84304278.9

(22) Date of filing: 25.06.84

(54) **Portable inducible control system, expression vectors containing them, microorganisms transformed with them, and their use in expressing exogenous protein.**

(30) Priority: 27.06.83 US 508388

(43) Date of publication of application:
02.01.85 Bulletin 85/01

(45) Publication of the grant of the patent:
06.02.91 Bulletin 91/06

(84) Designated Contracting States:
AT BE CH DE FR GB IT LI LU NL SE

(56) References cited:
EP-A-0 067 540     US-A-4 374 927
WO-A-84/04755

GENE, vol. 16, no. 1-3, December 1981 (Part II)
(Amsterdam) J.J. SNINSKY et al. "Construction
and characterization of a novel two- plasmid
system for accomplishing temperature-
regulated, amplified ex- pression of cloued
adventious genes in Escherichia coli" Pages
275-286

The file contains technical information
submitted after the application was filed and
not included in this specification

(73) Proprietor: GENENTECH, INC.
460 Point San Bruno Boulevard
South San Francisco California 94080 (US)

(72) Inventor: Henner, Dennis James
297 Angelita Avenue
Pacifica, CA 94404 (US)
Inventor: Yansura, Daniel George
330 Carmel Avenue
Pacifica, CA 94404 (US)

(74) Representative: Armitage, Ian Michael et al
MEWBURN ELLIS & CO. 2/3 Cursitor Street
London EC4A 1BQ. (GB)

Courier Press, Leamington Spa, England.

## Description

This invention relates to the field of producing foreign proteins in host bacteria using recombinant techniques. More specifically, the invention relates to novel control systems which regulate the expression of a desired gene and thus to production of its encoded protein in response to factors under the control of the experimenter. The invention is applicable to a wide range of gram positive bacteria as hosts.

It is now understood quite clearly that there is more to the successful use of recombinant techniques to produce desired proteins than merely inserting the appropriate gene into an expression vector and transforming a suitable host. Not only must the expression system be recognized by the host cell, but the timing of the expression must be regulated to insure that the protein is produced at high levels only when the cell can best tolerate the amounts of foreign protein. Foreign protein genes are often expressed at levels that produce protein in much greater amounts than those of any endogenous protein. If such large amounts of protein are the goal, they may be lethal, and expression is often best separated from growth phase. Alternatively, it may be desirable to regulate the specific level of protein production so as to optimize its functionality in the context of other cellular events. Accordingly, workers in this field have employed promoters which are susceptible to control by repressor binding to operator sequences either contained within the promoter sequence or slightly downstream.

Microorganisms themselves utilize several control mechanisms for regulating the level of protein production. In some organisms and for some proteins, control is exerted at the translational level by direct inhibition or stimulation of the rate of protein synthesis at the ribosome or by stabilization or destabilisation of mRNA. This translational approach at present does not easily lend itself to fine-tuned voluntary control by the experimenter, and has not been used to obtain the desired objectives of recombinant technology such as high levels of protein production. At least two control strategies have been described which are used by microorganisms at the level of transcription. One involves "sigma factors" which are produced by microorganisms at various stages of their life cycles, and which bind to RNA polymerase to render it more, or less, suitable for particular promoter sites in the DNA sequences to be transcribed. This method, like translational control, is not presently employed as a means to effect external control of expression, because it does not permit the desired level of controllability.

A second transcriptional strategy employs an "operator"—i.e., a sequence in the operon proximal to the promoter either included within the promoter itself or somewhat downstream, to which a repressor molecule is bound when the transcription is to be shut off. The repressor is removed from the operator in response to a depletion in its total effective amount, often obtained by supplying to the cell an inducer which inactivates the repressor protein. It is this strategy which has been co-opted by biotechnologists to effect their own control over expression. For example, see Sninsky, J. J., et al., Gene, 16: 275 (1981). However, heretofore, this approach has been available only in E. coli and closely related gram negative basis, since it is only in these systems that the operator/repressor mechanism has been described. Furthermore, such control systems have not been constructed in a form so that they can function in conjunction with any desired gene for expression in other hosts besides the natural host for the control system. The ability to so function renders the system "portable".

The present invention provides a gram position bacterium transformed for the expression of a subject coding sequence, the bacterium comprising:

(a) bacterial promoter/operator operably linked to the subject coding sequence so as to effect its expression in the bacterium, the operator being of gram negative bacterial origin; and

(b) a DNA sequence encoding a gram negative bacterial repressor of the operator of (a) under control of a bacterial promoter.

In another aspect the invention provides a method for the preparation of such a bacterium comprising transforming the bacterium with DNA containing elements (a) and (b). Thus, the invention concerns the provisions and use of a portable control system which comprises a promoter/operator sequence wherein the operator is potentially under the control of a repressor, plus the coding sequence for this repressor also operably linked to a suitable promoter. Such a compilation of sequences can be utilized in a large variety of hosts including hosts which are not known to employ such control systems endogenously. Thus a control system is provided which can be inserted into plasmid vectors or into the genome of the desired host organism so as to provide an inducible transcription control for a gene sequence operably linked to it. This is a suitable regulation system for genes encoding desired proteins which is easily manipulated and controlled, both in traditional E. coli hosts and more importantly, in less traditional, gram positive hosts. These latter hosts within their own comvlement of genetic and plasmid material either lack this system of controlling gene expression altogether or such systems of regulation are not yet known to be associated with them.

A preferred embodiment of the invention employs a hybrid promoter/operator comprising the sequence of the RNA polymerase recognition site for the penicillinase promoter and the operator region of the β-galactosidase (lac) promoter/operator, and a similar hybrid promoter comprising the sequence of the RNA polymerase recognition site of an SPO-1 phage promoter and the operator region of the lac promoter/operator. These hybrids are particularly effective as targets for repressor control

It is surprisingly for several reasons, in the context of present knowledge, that the control system of the invention is workable in a variety of hosts. It is known that the lacI repressor functions as a multimer in its usual *E. coli* millieu; it is uncertain whether the cellular environment of other organisms would permit the correct conformational arrangement so as to permit aggregation. Furthermore, the RNA polymerases of non-coliform hosts may be sufficiently different from that of *E. coli* to render the operator/repressor aggregation ineffective in inhibiting the polymerase. Finally, with respect to gram positives, it is unclear as to whether suitable inducers would be able to permeate into the cell to enable contact with repressor.

Brief description of the drawings

Figure 1 shows the construction of the shuttle vectors pBS42, pBSA42, and pBSA42 subclone.

Figure 2 shows the construction of the plasmid pBSA105 which contains the penicillinase gene under the control of the pac-1 promoter operator.

Figure 3 shows the construction of the plasmid pIQ45, an expression vector for the lacI repressor.

Figure 4 shows the construction of paIQ25, an expression vector for penicllinase under control of the portable system of the invention.

Figure 5 shows detection of penicillinase expression in IPTG induced cells grown on PVA plates.

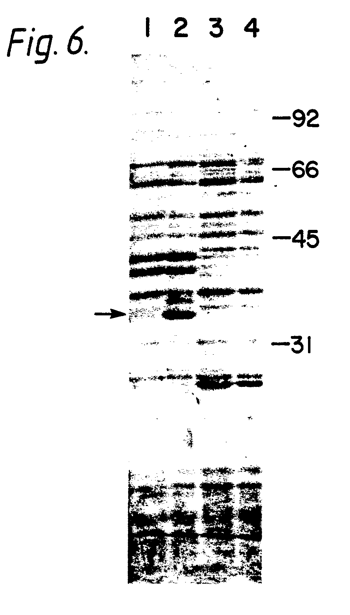

Figure 6 shows the results of SDS PAGE on supernatants derived from Bacillus cultures transformed with pAIQ25.

Figure 7 shows the construction of pLIQ1, an expression vector for leukocyte interferon A under the control system of the invention, and of pAIQ120.

Figure 8 gives the entire nucleotide sequence for each of the pac-1 and spac-1 hybrid promoter/operators.

Figure 9 shows the construction of pSPIF-III which contains the spac-1 promoter/operator operably linked to the human leukocyte interferon A gene.

Figure 10 shows the construction of pER which contains a chromosomal-complement insert.

Figure 11 shows the construction of pPCX-21 which contains the penicillinase gene under pac-1 control in an integrable plasmid.

Figure 12 shows the sequence of a fragment containing an Eco RI and a BstE II site used in the construction of pIQ45.

Detailed description

A. Definitions

As used herein, DNA sequences which are "operably linked" refers to DNA sequences which are juxtaposed in such a way that their respective functions are mutually dependent. For example, a promoter operably linked to a coding sequence is capable of effecting the expression of the coding sequence. An operator which is operably linked to a promoter is capable, when bound to a repressor protein, of inhibiting the function of the promoter, and when derepressed of permitting it to function normally. Such an operator sequence may overlap the promoter sequence or may lie downstream from it; "operably linked" is independent of such location as long as the functional interrelationship between the two sequences is maintained.

"Compatible repressor" meanspa protein which is capable of binding an operator sequence (to which it is compatible) whereby the operator is effective in inhibiting the promoter to which the operator is "operably linked". Thus, a "compatible" repressor has meaning only when referred to a related operator sequence.

"Inducible" promoter refers to a promoter which can be "turned on" by derepression in response to simple manipulations such as temperature shifts or addition of compounds to the medium harboring the organisms containing the promoter system, thus providing a voluntary system of expression. A typical example of such an "inducible" control system is placUV5 which contains a downstream operator sequence inducible by removing the "lac repressor". Such removal can be induced by adding isopropyl-β-D-thiogalactoside (IPTG) to the medium; the inducer permeates the cells, binds to repressor protein and thus permits expression of the β-galactosidase gene in untransformed cells or, in cells transformed by recombinant expression vectors of whatever gene is placed in control of the placUV5 promoter. This system is described in detail in U.S. Patent Application 264,306 filed May 18, 1981 and published in EPO publication No. 0067540, Dec. 22, 1982. Another commonly used promoter, whose inducibility, is however, less sensitive to additions to the medium, is the trp promoter, disclosed in U.S. Application Serial No. 133,296 filed March 24, 1980 and published in EPO publication No. 0036776. This promoter is inducible by addition of indole acrylic acid (IAA) to the culture medium. The inducer binds competitively with tryptophan to the repressor protein, but is less finely tuned to voluntary control than the aforementioned lac promoter because a minimal level of derepression is mandated by the required presence of tryptophan needed for protein synthesis.

"Portable" control system means that the system is capable of being ligated operably to a desired gene sequence, and capable of controlling expression in prokaryotic hosts in general—not just in the prokaryotic host of its origin. Such potential hosts include bacteria spanning the entire taxonomic scope of prokaryotes.

"Gram positive" bacteria refers to the standard definition for such bacteria wherein a single membrane envelope which permits active transport of substances into and out of the cell encloses the bacterium. Rigidity is supplied by a cell wall which is capable only of passive permeation. This class of organisms is important to the present invention because heretofore it has not been possible to provide inducible transcriptional controls in such organisms. The portable control systems of the present invention are, however, capable of providing such control.

On the other hand, *E. coli*, as representative of gram negative organisms—i.e., those which contain two enveloping membranes capable of active transport, are the traditional hosts for control systems analogous to those described above. Distantly related gram negative strains such as *Pseudomonas* are not known to possess such systems. Of course, a major advantage of the portable systems of the present invention is that they are useable in a wide variety of hosts. This does not mean, however, that they are inapplicable to the more commonly employed host systems such as *E. coli*.

## B. General description

The control system of the present invention employs recombinant techniques to splice together a promoter/operator and the gene (under suitable promoter regulation) encoding the compatible repressor which binds to the operator of the promoter/operator sequence. This package is then placed in operable functionality with a desired gene sequence. The control package therefore provides not only the promoter/operator under which the desired gene is to function, but also provides the expression system for the control mechanism, i.e., the repressor. While such provision is not mandatory for organisms such as *E. coli*, which contain genes capable of expressing repressor protein in their own genomes, it is a requisite part of the sequence where the host is to be an organism which does not encode the repressor.

A practical approach to the construction of the control system operably linked to DNA encoding a desired protein results in a tandem construction. The desired gene is placed under the control of promoter/operator, preferably a hybrid promoter/operator. The possibility of employing a hybrid promoter/operator permits the promoter to be selected from those which are known to be effective in an intended host organism, and the operator to be chosen from among those which are known to be controlled by a repressor capable of being affected by a compound introduced extracellularly i.e. an inducible operator. Thus, in one preferred embodiment, the penicillinase promoter which is known to be functional in *B. subtilis* is combined with the lac operator which is repressed by a protein affected in turn by a commonly used inducer, IPTG. The promoter/operator thus has features which permit its use in non-coliform hosts while permitting traditional coliform systems of control to be used. Either ligated to, or co-transfected with, the constructed hybrid promoter/operator/desired gene operon is an additional operon comprising a promoter compatible with the host cell operably linked to the gene encoding the repressor compatible with the promoter/operator of the extreme operon. A suitable combination here for use in, for example, *B. subtilis* is the penicillinase promoter operably linked to the gene encoding lacI repressor; however, any promoter operable in the desired host may also be used. Those promoters most commonly used in recombinant DNA construction include the β-lactamase (penicillinase) and lactose promoter systems (Chang et al, *Nature*, 275: 615 (1978); Itakura, et al, *Science*, 198: 1056 (1977); (Goeddel, et al *Nature* 281: 544 (1979)) and a tryptophan (trp) promoter system (Goeddel, et al, *Nucleic Acids Res.*, 8: 4057 (1980); EPO Appl. Publ. No. 0036776). While these are the most commonly used, other microbial promoters have been discovered and utilized, and details concerning their nucleotide sequences have been published, enabling a skilled worker to ligate them functionally with plasmid vectors or hybridize them to suitable operator sequences (Siebenlist, et al, *Cell* 20:269 (1980)).

Thus, the control system vector contains two complete operons, one a controllable operon for expression of the desired gene and the other permitting production of the control protein which is capable of repressing the promoter/operator which effects the desired gene expression.

In the preferred embodiments described hereinbelow, the desired gene sequences are the penicillinase gene of Bacillus and the gene encoding leukocyte interferon. Of course, by use of suitable recombinant techniques, any desired gene may be placed under the control of the system of the invention.

Further, the constructions, for convenience, contain all elements of the control system as well as the desired gene on the same expression vector. Such constructions are only one example of the manner in which the invention may be practiced. It is also possible to place, for example, the promoter/operator/desired gene system on one plasmid, and the repressor expression system on another, and to cotransform suitable hosts with both plasmids. Also, by using complementary sequences on the plasmids constructed to those found in the host genome, an enhanced integration of the plasmid sequences into the genome of the host may be effected. Accordingly, the control systems of the present invention may be found not only in portable vector constructions, but also integrated into the genetic material of the transformed host.

## C. Methods employed
### C.1. Vector construction

Construction of suitable vectors containing the desired coding and control sequences employ standard ligation techniques. Isolated plasmids or DNA fragments are cleaved, tailored, and religated in the form desired to form the plasmids required. The methods employed are not dependent on the DNA source, or on intended host.

Cleavage is performed by treating with restriction enzyme (or enzymes) in suitable buffer. In general, about 1 μg plasmid or DNA fragments is used with about 1 unit of enzyme in about 20 μl of buffer solution. (Appropriate buffers and substrate amounts for particular restriction enzymes are specified by the manufacturer). Incubation times of about 1 hour at 37°C are workable. After incubations, protein is removed by extraction with phenol and chloroform, and the nucleic acid is recovered from the aqueous fraction by precipitation with ethanol.

If blunt ends are required, the preparation is treated for 15 minutes at 15° with 10 units of *E. coli* DNA Polymerase 1 (Klenow), phenol-chloroform extracted, and ethanol precipitated.

Size separation of the cleaved fragments is performed using 6 percent polyacrylamide gel described in Goeddel, D. , et al., *Nucleic Acids Res.*, 8: 4057 (1980) incorporated herein by reference.

For ligation, approximately equimolar amounts of the desired components, suitably end tailored to provide correct matching are treated with about 10 units T4 DNA ligase per 0.5 µg DNA. (When cleaved vectors are used as components, it may be useful to prevent religation of the cleaved vector by pretreatment with bacterial alkaline phosphatase).

In the examples described below correct ligations for plasmid construction are confirmed by transforming *E. coli* K12 strain 294 (ATCC 31446) with the ligation mixture or other suitable microorganisms such as B. subtilis, strain 1168. Other *E. coli* strains used for the constructions were D1210 (Sadler, J. R. *et al*, 1980 Gene 8; 279) which was used for the construction of plasmids containing hybrid promoters and strain 3300 (*E. coli* Genetic Stock Center No. 808) which was used for the construction of plQ45. Successful transformants were selected by ampicillin, tetracycline, chloramphenicol or neomycin resistance depending on the mode of plasmid construction. Plasmids from the transformants were then prepared, analyzed by restriction and/or sequenced by the method of Messing, et al, *Nucleic Acids Res.*, 9: 309 (1981) or by the method of Maxam, et al, *Methods in Enzymology*, 65: 499 (1980).

A commonly used technique for obtaining a DNA sequence cleaved at a specific location, which is used frequently in the invention, is the "primer repair" reaction described in US 133,296, filed March 4, 1980, incorporated by reference, and published as EPO Application Publication No. 0036776 September 30, 1981. In this reaction, the fragment of DNA desired to be specifically cleaved is denatured and mixed with a primer approximately 12 bases long, which is complementary to one of the denatured strands. The primer is constructed so that one end of the primer is exactly contiguous with the desired cleavage point. The primer can be designated so as to be complementary either to the sense or anti-sense strand, thereby controlling the direction of "repair". In the repair, the mixture is treated with DNA polymerase I (Klenow fragment) which effects the synthesis of a strand complementary to the denatured, primer bound, DNA in the 5' to 3' direction starting as the 3' end of bound primer, and cleaving back the remaining single stranded denatured portion extending from the primer's 5' end.

## C.2. Transformation

Cell transformation of *E. coli* was accomplished by the $CaCl_2$ treatment of Cohen, F.N. et al., *Proc., Natl. Acad. Sci. (USA)*, 69: 2110 (1972). Transformation of B. subtilis employed the method of Anagnostopoulos, C., et al., *J. Bacteriol.*, 81: 741 (1961). Selection for successful transformants using the plasmids of the present invention was conducted by suitable concentrations of the appropriate antibiotic: 12.5 µg/ml chloramphenicol (CMP). 20 µg/ml neomycin (NEO), 20 µg/ml ampicillin (AMP), 5 µg/ml erythromycin (ERY), and 3 µg/ml tetracycline (TET).

## C.3. Assays

Penicillinase is assayed (including detection of activity on polyvinylalcohol (PVA) plates) by the method of Sherratt, D. J. et al., *J. Gen. Microbiol.*, 76: 217 (1973). Leukocyte interferon assays were performed by growing B. subtilis strain I168 that had been transformed with the described plasmids in L. broth supplemented with 10 µg/ml NED and 0.5 percent glucose at 37°C until the cells attained a density at OD 600 of 1.0. Triplicate one ml aliquots were harvested by centrifugation in an Eppendorf microfuge for 4 min. The pellets were suspended in 0.1 ml of a solution of 10 mg/ml lysozyme in 10 mM Tris, 1 mM EDTA, pH 8 and incubated at 37°C for 15 min. 0.9 ml of 0.1 percent SDS was then added and the cells were diluted 50—300 fold in phosphate buffered saline containing 1 mg/ml bovine serum albumin. Interferon levels were determined by the cytopathic effect inhibition assay using vesicular stomatitis virus on MDBK cells as described by Stewart, W. E. *The Interferon System*, Springer, Berlin (1979).

## D. Detailed description of preferred embodiments
### D.1. Controlled expression of B. licheniformis penicillinase
#### D.1.1 Construction of the expression vector

The plasmid pAlQ25 contains the penicillinase gene operably linked to the control system of the present invention. In this construction, the penicillinase gene is preceded by a hybrid promoter/operator comprising a portion of the penicillinase promoter and the lac operator. Downstream from the penicillinase gene is the coding sequence for the lacI repressor protein under the control of the penicillinase promoter. The backbone portions of this plasmid contain an origin of replication operable in Bacillus.

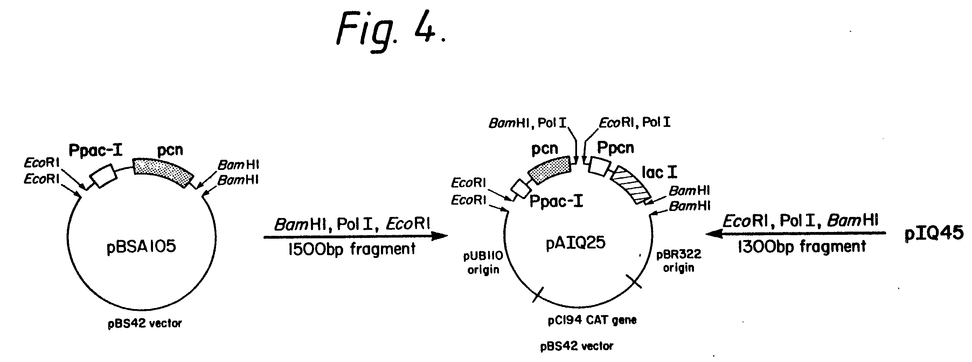

This plasmid is constructed by ligation of three fragments (see Figure 4). Fragment 1 is a 1500 base pair EcoR1-blunt ended fragment derived from an intermediate plasmid pBSA105, which contains the hybrid (pac-1) promoter operably linked to the penicillinase gene. Fragment 2 is a 1300 base pair blunt ended-BamH1 fragment derived from intermediate plasmid plQ45 which contains the lacI gene under the control of the penicillinase promoter. Fragment 3 is an EcoR1-BamH1 backbone fragment derived from pBS42.

Construction of the three fragments used in this ligation is described below.

D.1.2 Construction of fragment 1—Penicillinase operon containing the Pac-1 promoter/operator

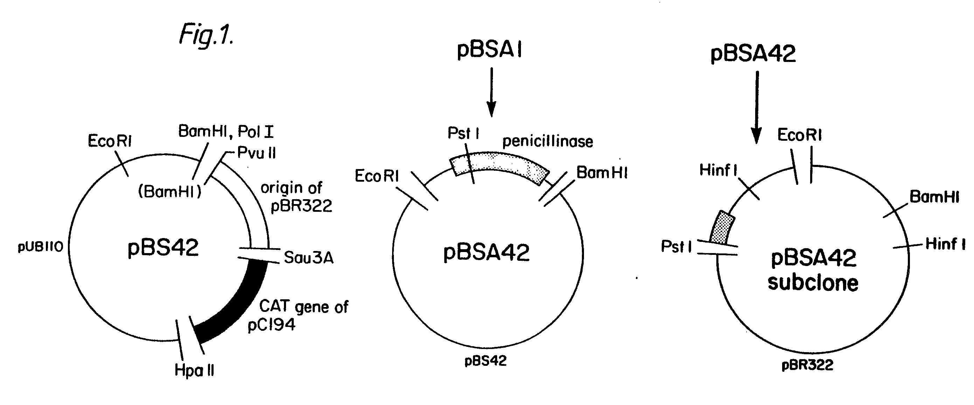

Fragment 1, containing the pac-1 promoter/operator and the penicillinase gene is derived from an intermediate plasmid pBSA105 by treating first with BamH1, filling in using the Klenow fragment of DNA polymerase, followed by digestion with EcoR1. pBSA105 is constructed as follows: In step 1 the penicillinase gene is inserted into the pBS42 vector described in paragraph D.1.4 below to give pBSA42 (see Figure 1). The penicillinase gene of *Bacillus licheniformis* strain −749/C (ATCC 25972) was isolated by the method of Imanaka, T. et al. *J. Bacteriol.* 147: 774 (1981). The vector plasmid used in the isolation of the penicillinase gene was pBS7 which is described in paragraph D.3.1. The pBS7 derivative carrying the penicillinase gene was designated pBSA1.

Step 1 was accomplished by opening pBS42 by double digestion with EcoR1 and BamH1, and religating in the presence of the entire penicillinase gene which had been modified by converting a HpaII site 40 base pairs upstream of the promoter into an EcoR1 site, and the PvuII site 600 base pairs downstream from the 3′ end of tys gene to a BamH1 site. These conversions employed standard techniques. The resultant pBSA42 plasmid then contains the entire penicillinase gene inserted into a pBS42 backbone. A subclone of pBSA42 (pBSA subclone) was constructed by ligating the EcoRI-PstI fragment of pBSA42 containing the penicillinase promoter into an EcoRI-PstI digested pBR322 vector (Figure 1). pBSA42 or its subclone provided the three fragments, A, B, and C which were ligated to provide pBSA105.

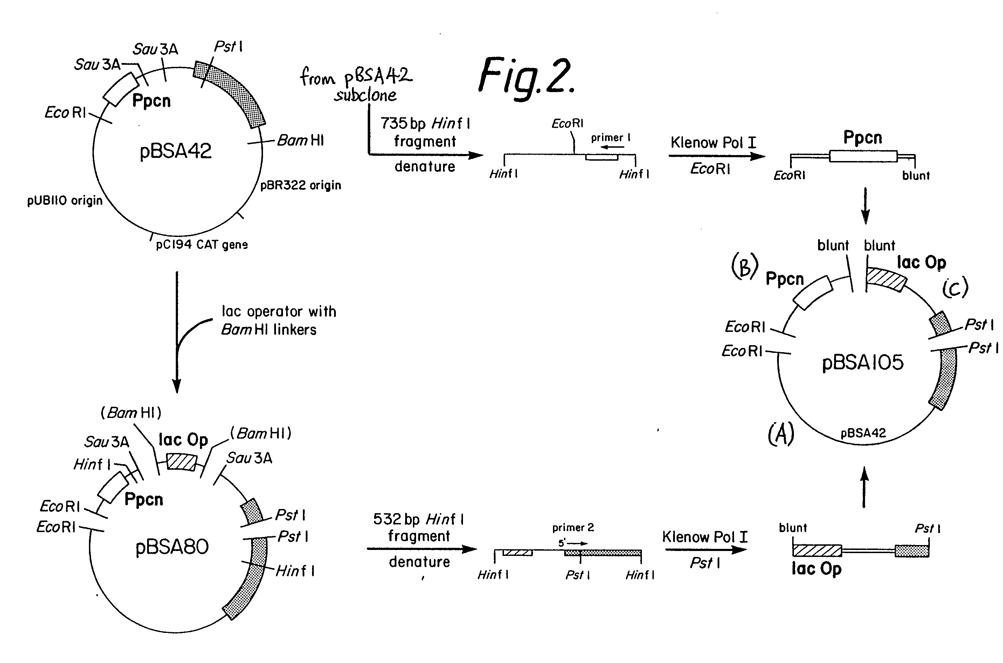

Fragment A is the product of double digestion of pBSA42 with EcoR1 and Pst1 as shown in Figure 2. This provides the backbone portion of pBSA42 along with the C-terminal portion of the penicillinase gene.

Fragment B contained a modified penicillinase promoter and is derived from a subclone of pBSA42 by Hinfl cleavage. As shown in Figure 2, digestion of the pBSA42 subclone with Hinfl provides a 730 base pair fragment which contains the penicillinase promoter (as well as a portion of the gene). The Hinfl cleavage fragment is then denatured and subjected to a primer repair reaction so as to cut back to position −4 in the penicillinase promoter. The primer used is 5′-GAAAGTATTAC-3′ which binds to the DNA beginning at approximately −4 reading from the 33 direction. The resulting blunt ended fragment is further cleaved at the EcoR1 site upstream from the promoter. The resulting fragment B thus contains that portion of the penicillinase promoter which is upstream of position −4.

Fragment C contains the lac operator and the N-terminal portion of the penicillinase gene. To form fragment C, an intermediate plasmid pBSA80 is constructed from pBSA42 by insertion of the lac operator. pBSA42 was digested with Sau3A which deletes an 80 base pair fragment between the promoter and ribosome binding site. A synthetic DNA sequence containing the lac operator bounded by BamH1 sites, i.e., having the sequence

5′-GATCCGGTGTGGAATTGTGAGCGGATAACAATTCCG
GCCACACCTTAACACTCGCCTATTGTTAAGGCCTAG-5′

was then inserted by ligation into the remaining Sau3A sites, to give pBSA80.

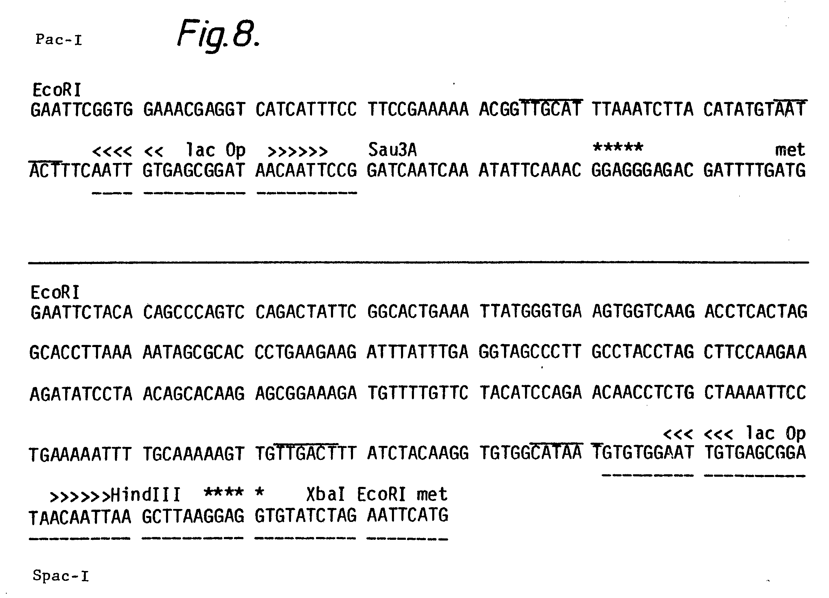

This intermediate plasmid pBSA80 was then treated to form fragment C. Treatment with Hinfl yields a 532 base pair fragment which contains the penicillinase ribosome binding site, the entire lac operator, and the N terminal portion of the penicillinase gene. A primer repair reaction of this fragment serves to remove the RNA polymerase binding site. The primer used was 5′ AATTGTGAGCGG-3′ which binds at the immediate 5′ end of the lac operator in this construction. The resulting shortened fragment was further cleaved with Pstl to obtain the shortened N-terminal portion of the gene. Fragment C contains pair of the hybrid pac-1 promoter/operator. The sequence of this promoter/operator is shown in Figure 8.

Fragments A, B, and C were then ligated to provide the desired plasmid pBSA105. This plasmid contains the hybrid pac-1 promoter/operator which includes a portion of the pencillinase promoter and the lac operator both upstream from the penicillin ribosome binding site and penicillinase gene.

D.1.3 Construction of fragment 2 containing the Lacl repressor gene under control of the penicillinase promoter

Fragment 2 is the 1300 base pair fragment derived from plQ45 by digestion with EcoR1, blunt ending with DNA polymerase, and followed by treatment with BamH1. plQ45 is constructed of fragments from a subclone of pBSA42 as described above, and pHiQ6, described in Hare, D.L. et al, *Gene*, 3: 269 (1978). pHiQ6 contains the entire lacl gene.

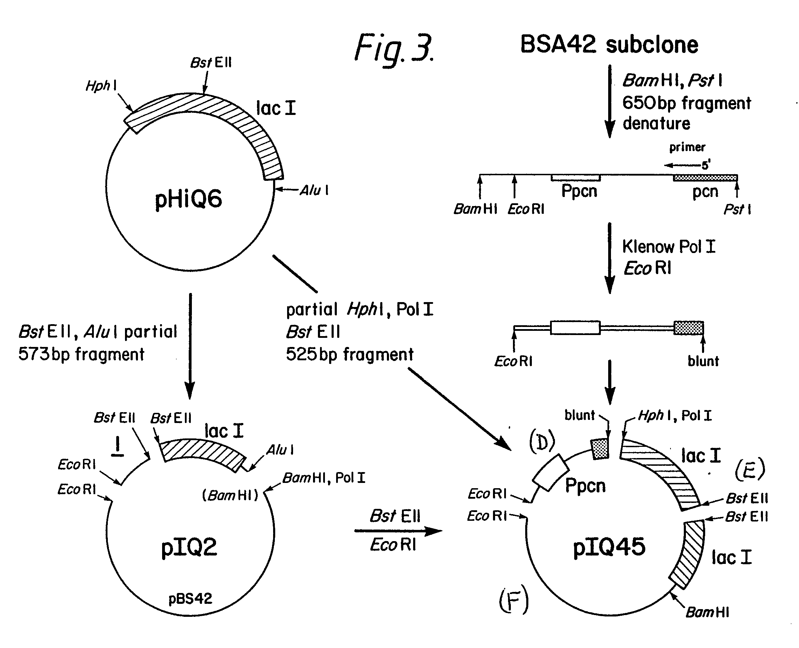

In the three way ligation which forms plQ45, fragment D is derived from pBSA42 and contains the penicillinase promoter and the first two amino acids of the penicillinase gene. Since the first two amino acids of the penicillinase gene are identical to the first two amino acids of the lacl gene, fragment D can usefully be used in the reconstruction of the lacl gene coding sequence. To construct fragment D, the pBSA42 subclone is digested with BamH1 and Pst1 to provide a 650 base pair fragment containing the penicillinase promoter and the N-terminal portion of the penicillinase gene. The gene is shortened to include only the codons for the first two amino acids by a primer repair reaction using as primer against the denatured negative sense strand of this fragment, 5′-TTTCATCAAAA-3′. The resulting abbreviated fragment was further cleaved with EcoR1 to give fragment D as shown in Figure 3.

Fragments E and F are ultimately derivable from pHiQ6 as follows: Fragment E is formed by a partial digestion of pHiQ6 with Hph1, blunt ending with polymerase 1, and followed by treating with BstEll and isolating the 525 base pair fragment which contains the N-terminal portion of the lacl gene beginning at amino acid number 3. Thus, fragments D and E together will supply the promoter and the N-terminal portion of the gene preceding the BstEll site.

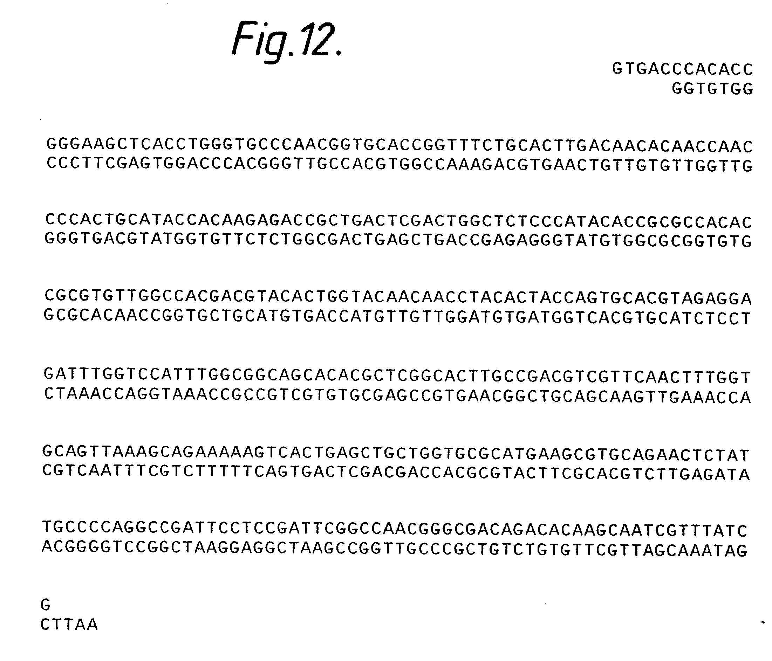

The remaining portion of the gene and the backbone portion of plQ45 is supplied by fragment F. Fragment F is derived from pHiQ6 through an intermediate plasmid, plQ2 which includes the backbone portion of pBS42. This plasmid is formed by a three-way ligation between a BstEll-partial Alu 1 double digestion of pHiQ6 (see figure 3) the EcoR1 filled-in BamHI fragment from pBS 42 (see Figure 1) and a fragment containing an ECoR1 and BstEll site which has the base sequence shown in Figure 12. Ligation of these three fragments results in plQ2 which contains the pBS42 backbone, and the portion of the lacl gene extending from the BstEll site to the C terminus. Cleavage of plQ2 with BstEll and EcoR1 provides fragment F.

The plasmid plQ45 resulting from ligation of fragments D, E and F therefore contains the penicillinase promoter linked to the entire lacl gene sequence.

D.1.4 Construction of fragment 3-backbone segment

Fragment 3 is the vector portion of pBS42 double digested with EcoR1 and BamH1. pBS42 is formed by three way ligation of fragments derived from pUB110, pC194, and pBR322 (see Figure 1). The fragment from pUB110 is the approximately 2600 base pair fragment between the HpaII site at 1900 and the BamH1 site at 4500 and contains an origin of replication operable in Bacillus: Grycztan, T. J. et al., *J. Bacteriol.*, 134: 318 (1978); Jalanko, A. et al., *Gene,* 14: 325 (1981). The BamHI site was blunt ended by filling in using DNA polymerase I. The pBR322 portion is the ~1100 base pair fragment between the PvuII site at 2067 and the Sau3A site at 3212 which contains the *E. coli* origin of replication: Bolivar, F. et al., *Gene* 2: 95 (1977); Sutcliffe, J. G. *Cold Spring Harbor Symposium* 43: I, 77 (1978). The pC194 fragment is the ~1200 base pair fragment between the HpaII site at 973 and the Sau3A site at 2006 which contains the gene for chloramphenicol resistance expressible in both *E. coli* and *B. subtilis*. Ehrlich, S. D. *Proc., Natl. Acad. Sci.* (USA), 74: 1680 (1977); Horynuchi, S., et al., *J. Bacteriol.* 150: 815 (1982).

The resulting plasmid pBS42 thus contains origins of replication operable both in *E. coli* and in Bacillus and an expressable gene for chloramphenicol resistance. Since the ligation recreatres the BamH1 site derived from pUB110, double digestion of pBS42 with EcoR1 and BamH1 provides substantially the entire plasmid.

D.1.5 Expression of the Penicillinase Gene

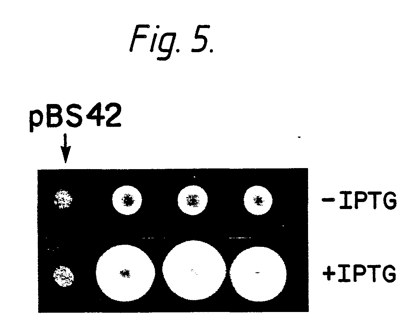

The ligation mixture containing pAIQ25 (see D.1.1) was transformed into *Bacillus subtilis*, strain Bacillus Genetic Stock Center, Columbia, Ohio, No. 1A1 (ATCC No. 27689) and successful transformants selected by chloroamphenicol resistance. Several of these were picked to replicate on PVA indicator plates (see paragraph C.3), with and without ImM IPTG. The results, shown in Figure 4, indicate that production of penicillinase was enhanced in the presence of IPTG. Representative colonies were grown overnight in LB +0.5 percent glucose and 10 µg/ml chloramphenicol with and without 1 mM IPTG. Appropriate dilutions of the cell broth in 0.1M sodium phosphate buffer (pH 7.0) were assayed for penicillinase by the method of Sherratt et al. (supra at C.3). Cells grown in the presence of IPTG showed penicillinase levels at an average of 6,000 units/ml of cell broth while those grown in the absence of IPTG produced only 60 units/ml of culture. Further, the supernatant fraction of these cultures when subjected to SDS-PAGE showed a band of 33,000 molecular weight (the approximate MW of penicillinase) only in cultures induced by IPTG (figure 5).

In Figure 5 Lane 1 shows I168 transformed with pAIQ25, grown without IPTG and Lane 2, I168 transformed with pAIQ25, grown with IPTG, Lanes 3 and 4, I168 transformed with pBS42, grown with and without IPTG.

D.2 Construction of the spac-I promoter

The entire sequence of the spac-I hybrid promoter is shown along with that of the pac-I hybrid in Figure 3. The sequence that is not underlined corresponds to the natural sequence of the SPO-1 promoter, the underlined sequence corresponds to the lac operator (designed on the figure) and a Shine-Dalgarno sequence (designated with *'s).

The spac-I promoter is similar to the pac-I promoter whose construction is described in D.1.2 above, except that the RNA polymerase recognition site is derived from a B. subtilis phage promoter, the sequence of which is known to correspond to that which lies on the EcoR1* fragment 26 of SPO-1 DNA as described by Lee, G. et al., *Mol. Gen. Genet.* 180: 57 (1980). SPO-1 DNA was prepared as described by Lee, G., et al. (supra) and EcoR1* fragment 26 was prepared by digestion of the SPO-1 DNA with 10 U/ug in 10 percent glycerol, 0.025 M Tris-Hcl pH 8.5, and 0.002 µgC12, fractionation on a 5 percent acrylamide gel, and electroelution of the 1.1 kbp fragment 26. The previous reported sequence showed that a HindII site cut within the −35 portion of the RNA polymerase recognition site and the 232 bp fragment contained a part of the −35 sequence and the 5' "upstream" region was isolated. Synthetic DNA which recreated the remainder of the RNA polymerase recognition site was ligated onto the 232 bp fragment. This sequence extended to the end of the −10 portion of the recognition site. Further synthetic DNA which contained the lac operator and a Shine-Dalgarno sequence (or ribosome binding site) was ligated to the recreated RNA polymerase recognition site.

D.3 Controlled expression of the leukocyte interferon A gene.

D.3.1 Construction of pLIQ1

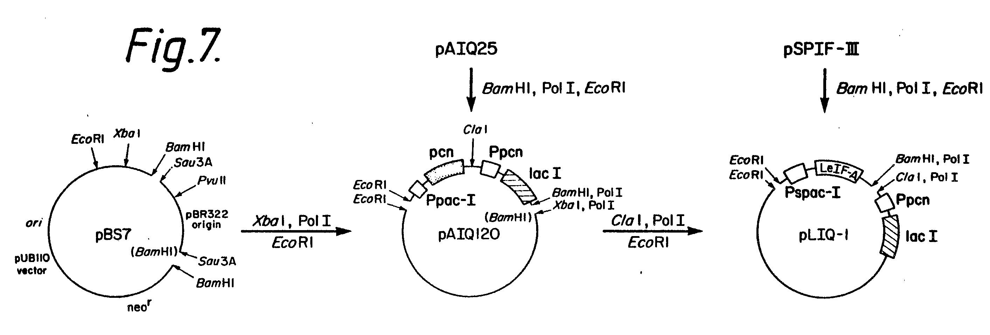

Figure 7 shows the construction of pLIQ1. pLIQ1 is formed by a two way ligation of a fragment from plasmid pAIQ120 and pSPIF-III which contains the spac-1 hybrid promoter.

Plasmid pAIQ120 is analogous to the previously described plasmid pAIQ25, the major difference is that the backbone vector is a neomycin resistant plasmid. The construction of pAIQ120 is shown in Figure 7.

The parent plasmid pBS7 is a derivative of the plasmid pUB110 described by Grycztan, T. J. et al., *J. Bacteriol.* 134: 318 (1978) which has been digested with BamHI, and a partial Sau3A fragment containing the origin region of pBR322 ligated into the BamHI site. The Sau3A fragment extends from the Sau3A site at 1666 to the Sau3A site at 2332 on the standard pBR322 map (as in New England Biolabs catalogue). The BamHI site indicated in parenthesis was recreated in the ligation. pBS7 is digested with Xba 1, blunt-ended with Klenow, and treated with EcoR1. The resulting fragment is ligated with the fragment resulting from BamHI digestion, blunt ending, and EcoRI digestion of pAIQ25 to give pAIQ120.

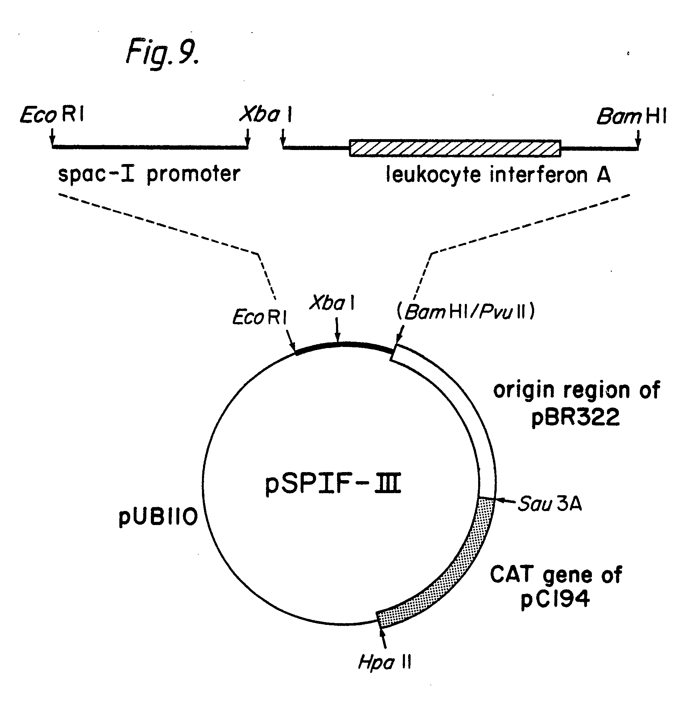

The construction of pSPIF-III is shown in Figure 9. Three fragments of DNA were ligated. The first was the spac-1 promoter from the EcoRI site to the XbaI site as indicated in Figure 8. The second was the leukocyte interferon A gene from the XbaI site preceding the initiation codon to a BamHI site past the gene in the pBR322 vector plasmid. This fragment was isolated from a derivative of plasmid pLeIFA25 described by Coeddel et al. (1980) *Nature 287:* 411. This derivative has the leukocyte interferon gene placed on the pBR322 vector plasmid between the EcoRI and BamHI sites rather than between the EcoRI and PstI sites and was constructed by standard techniques. The nucleotide sequence between the XbaI site and the initiation codon of the interferon gene is identical to that of pLeFA25 described above. The third consisted of the backbone portion of the pBS42 which had been digested with EcoRI and BamHI. To make pLIQ1, pSPIF-III is digested with BamHI blunt ended with polymerase and treated with EcoR1, and the fragment containing the leukocyte interferon gene and the spac-1 promoter isolated. Digestion of pAIQ120 with Cla 1, blunt ending with polymerase and treatment with EcoR1 provides a backbone fragment containing the lacI gene under control of the penicillinase promoter and compatible with the pSPIF-III fragment. Ligation of these fragments provides the desired plasmid, pLIQ1, as shown in Figure 7.

D.3.2 Production of Leukocyte Interferon under Inducible control

PLIQ1 was transformed into *B. subtilis* strain I168 and successful transformants selected by neomycin resistance. Successful transformants were grown in shake flasks containing LB+0.5 percent glucose+10 µg/ml neomycin and in the presence or absence of 1 mM IPTG. The cultures were assayed for leukocyte interferon by the method described in paragraph C.3. Cultures grown in the presence of IPTG gave levels of interferon of 100,000 units/ml (at 10D 600) while culture grown in the absence of IPTG showed levels of 2000 units/ml/OD 600.

Use of constructions analogous to those in Examples D.1 and D.3 but substituting for penicillinase or leukocyte interferon other genes such as genes for proinsulin, or β- or γ-interferons is well within the skill of the art. The plasmids exemplified herein may be cleaved with suitable restriction enzymes to excise the portions of DNA coding for penicillinase or leukocyte interferon A and these segments replaced by ligation with DNA fragments encoding desired proteins.

D.4 Integration of control sequences into the host genome

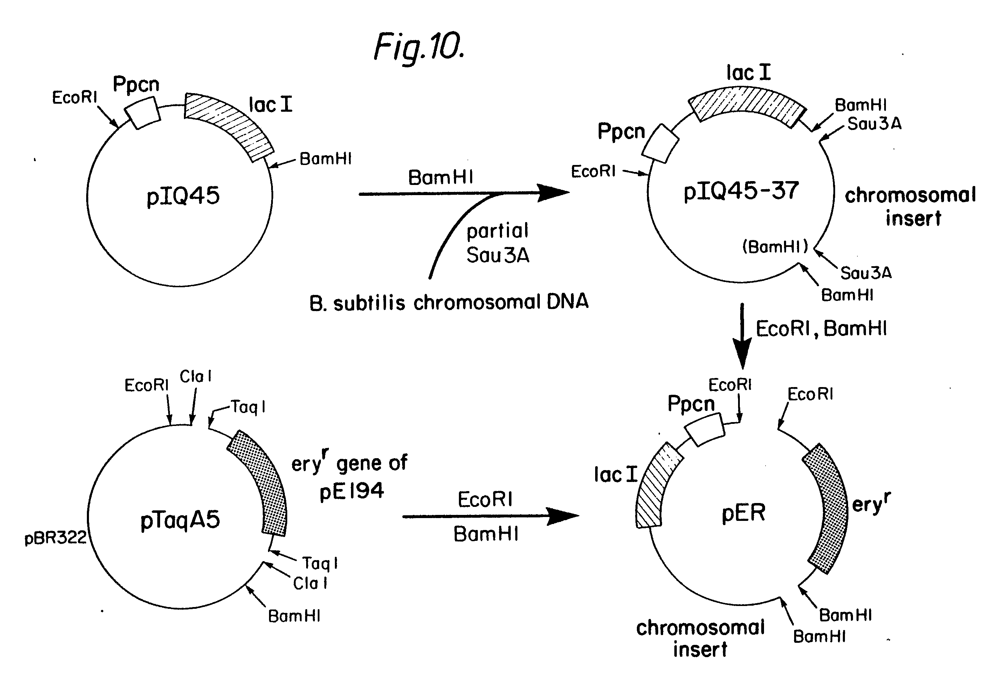

The repressor plasmid pIQ45 described in D.1.3, and containing the repressor gene, was digested with BamHI and ligated with B. subtilis DNA which had been partially digested with Sa3A. B. subtilis DNA was prepared by the method of Lovett, P.S., et al, *Methods in Enzymology*, 68: 342 (1979), except that proteinase K was substituted for pronase. Cmp^r transformants were selected and their plasmids analyzed by restriction digestion. A derivative of pIQ45 which had a Sau3A insert and a unique BamHI site on the site of the insert distal to the lac repressor gene was chosen and designated pIQ45-37 (Figure 10).

The erythromycin resistance gene from plasmid pE194 which was described and seqeunced by Horinouchi, S. and Weisblum, B. *J. Bacteriol.* 150:804 (1982) was used as the selectable marker for introducing the lac repressor into the B. subtilis chromosome. In order to create useful restriction sites, the largest TaqI fragment of pE194, which contains the erythromycin resistance gene, was isolated and ligated into the ClaI site of pBR322. This derivative, called pTaqA5, contains the ery resistance gene flanked by the EcoRI and BamHI sites of pBR322.

pTaqA5 was digested with EcoRI and BamHI and the fragment containing the ery resistance gene was isolated. pIQ45-37 was also digested with EcoRI and BamHI and the fragment containing the lac repressor gene and fragment of the B. subtilis chromosome was isolated. These two fragments were ligated and transformed into B. subtilis I 168.

Since neither of these two fragments contains an origin of replication, the only way that ery resistant transformants can arise is through recombination of the plasmid into the chromosome, a process previously described by Haldenwang, W. G., et al., *J. Bacteriol.* 142: 90 (1980).

Since the lac repressor was covalently linked to the piece of chromosomal DNA needed for the integration of the ery resistance gene, all the ery resistant transformants should have the lac repressor also integrated into the chromosome. One particular ery resistant transformant was chosen and designated I168:ER.

D.4.2 Integration of the pac-I controlled penicillinase into I168:ER

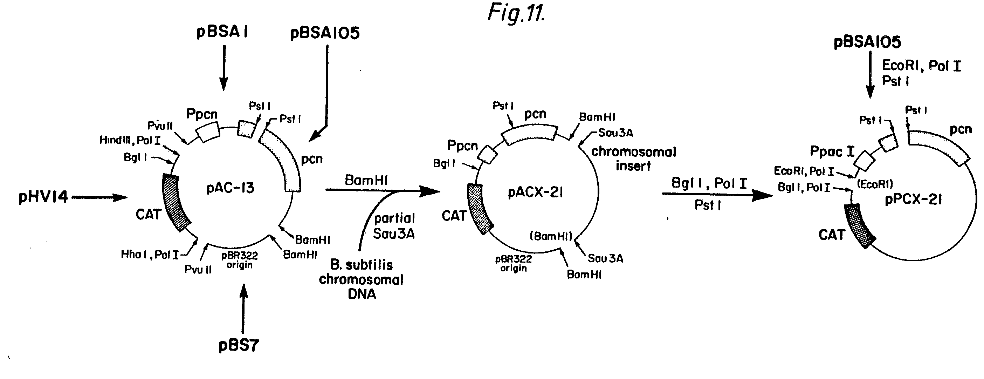

The construction of pACX-21 carrying the pac-I controlled penicillinase gene which will integrate into the B. subtilis chromosome is shown in Figure 11. The parent plasmid pAC13, was constructed via a four fragment ligation as shown in Figure 11.

The first PvuII-PstI fragment contains the natural penicillinase promoter and the front portion of the penicillinase gene and was derived from pBSA-1. The second Pst1-BamHI fragment contains the back portion of the penicillinase gene and is derived from pBSA-105. The third PvuII-BamHI fragment contains the pBR322 origin of replication and derived from plasmid pBS42. The fourth HindIII-HhaI fragment contains the chloramphenicol resistance (CAT) gene from plasmid pC194 and was isolated from plasmid pHv14 described by Enrlich, S. D., *Proc. Nat. Acad. Sci.* USA 75: 1433 (1978).

In the same manner as described in the paragraph above, random Sau3A fragments of B. subtilis chromosomal DNA were inserted into the unique BamHI site of pAC13. One particular derivative was chosen which lacked any EcoRI, PstI or BglI sites in the random insert and was called pACX-21 (Figure 11).

The penicillinase gene of pACX-21 was placed under the control of the pac-I promoter by simply replacing the natural promoter with the pac-I promoter from pBSA105 to give pPCX-21 (Figure 11).

Plasmids pPCX-21 and pACX-21 were transformed into B. subtilis strains I168:ER and selected for the integration of the plasmid by CMP resistance. Penicillinase assays of each plasmid integrant were performed as previously described. The results are shown below.

|          | −IPTG     | +IPTG     |
|----------|-----------|-----------|
| pACX-21  | 3500 U/ml | 3700 U/ml |
| pPCX-21  | 160 U/ml  | 3800 U/ml |

**Claims**

1. A gram positive bacterium transformed for the expression of a subject coding sequence, the bacterium comprising:
   (a) a bacterial promoter/operator operably linked to the subject coding sequence so as to effect its expression in the bacterium, the operator being of gram negative bacterial origin; and
   (b) a DNA sequence encoding a gram negative bacterial repressor of the operator of (a) under control of a bacterial promoter.

2. A transformed bacterium of Claim 1 wherein the operator is the lac operator and the repressor is lac I.

3. A transformed bacterium of Claim 1 wherein (a) comprises the DNA sequence of the *B. Licheniformis* penicillinase promoter RNA polymerase recognition site and the operator region of the *E. coli* lac promoter/operator.

4. A transformed bacterium of Claim 1 wherein the promoter/operator of part (a) is a hybrid in which the promoter is selected from among those which are known to be effective in the host organism and the operator is an inducible operator.

5. A transformed bacterium of Claim 4 wherein (a) comprises the DNA sequence of the SPO-1 phage promoter RNA polymerase recognition site and the operator region of the *E. coli* lac promoter/operator.

6. A transformed bacterium of Claim 1 wherein the operator and repressor are an *E. coli* operator and repressor.

7. A bacterium of any one of the preceding claims transformed with a vector which contains both the subject coding sequence gene (a) and the repressor gene (b).

8. A transformed bacterium of any one of the preceding claims wherein the subject coding sequence gene (a) and the repressor gene (b) have been incorporated into the host genome.

9. A method for the preparation of a gram positive bacterium transformed for the expression of a subject coding sequence, the bacterium comprising:
   (a) a bacterial promoter/operator operably linked to the subject coding sequence so as to effect its expression in the bacterium, the operator being of gram negative bacterial origin; and
   (b) a DNA sequence encoding a gram negative bacterial repressor of the operator of (a) under control of a bacterial promoter;
   said method comprising transforming the bacterium with DNA containing elements (a) and (b).

10. A method according to Claim 9 wherein the transforming DNA containing elements (a) and (b) is transferred to said bacterium from a different bacterial host.

11. A method according to Claim 10 wherein the transforming DNA is transferred from a gram negative bacterial host.

12. A method of any one of Claims 9 to 11 wherein the operator is the lac operator and the repressor is lac I.

13. A method of any one of Claims 9 to 11 wherein (a) comprises the DNA sequence of the *B.*

9

*Licheniformis* penicillinase promoter RNA polymerase recognition site and the operator region of the *E. coli* lac promoter/operator.

14. A method of any one of Claims 9 to 11 wherein the promoter/operator of part (a) is a hybrid in which the promoter is selected from among those which are known to be effective in the host organism and the operator is an inducible operator.

15. A method of Claim 14 wherein (a) comprises the DNA sequence of the SPO-1 phage promoter RNA polymerase recognition site and the operator region of the *E. coli* lac promoter/operator.

16. A method of any one of Claims 9 to 11 wherein the operator and repressor are an *E. coli* operator and repressor.

17. A method of any one of Claims 9 to 16 wherein the transformation is carried out with a vector which contains both the subject coding sequence gene (a) and the repressor gene (b).

18. A method of any one of Claims 9 to 17 wherein the subject coding sequence gene (a) and the repressor gene (b) are incorporated into the host genome.

**Patentansprüche**

1. Grampositives Bakterium, das für die Expression einer gegenständlichen Kodierungssequenz transformiert ist, das Bakterium umfassend:

(a) einen bakteriellen Promotor/Operator, der operabel an die gegensändliche Kodierungssequenz gebunden ist, um ihre Expression im Bakterium zu bewirken, wobei der Operator gramnegativen Ursprungs ist; und

(b) eine DNA Sequenz, die einen gramnegativen bakteriellen Repressor des Operators von (a) unter der Steuerung eines bakteriellen Promotors kodiert.

2. Transformiertes Bakterium nach Anspruch 1, worin der Operator der lac Operator und der Repressor lac I ist.

3. Transformiertes Bakterium nach Anspruch 1, worin (a) die DNA Sequenz der B. licheniformis Penicillinasepromotor RNA Polymerase-Erkennungsstelle und den Operatorbereich des E. coli lac Promotor/Operators enthält.

4. Transformiertes Bakterium nach Anspruch 1, worin der Promotor/Operator von Teil (a) ein Hybrid ist, in dem der Promotor und jeden ausgewählt wird, von denen bekannt ist, daß sie im Wirtsorganismus wirksam sind, und der Operator ein induzierbarer Operator ist.

5. Transformiertes Bakterium nach Anspruch 4, worin (a) die DNA Sequenz der SPO-1 Phage-Promotor-RNA-Polymerase-Erkennungsstelle und den Operatorbereich des E. coli lac Promotor/Operators enthält.

6. Transformiertes Bakterium nach Anspruch 1, worin der Operator und Repressor ein E. coli Operator und Repressor ist.

7. Bakterium nach einem der vorhergehenden Ansprüche, das mit einem Vektor transformiert ist, der sowohl das gegenständliche Kodierungssequenzgen (a) als auch das Repressorgen (b) enthält.

8. Transformiertes Bakterium nach einem der vorhergehenden Ansprüche, worin das gegenständliche Kodierungssequenzgen (A) und das Repressorgen (b) in das Wirtsgenom einverleibt sind.

9. Verfahren zur Herstellung eines grampositiven Bakteriums, das für die Expression einer gegenständlichen Kodierungssequenz transformiert ist, das Bakterium umfassend:

(a) einen bakteriellen Promotor/Operator, der operabel an die gegenständliche Kodierungssequenz gebunden ist, um ihre Expression im Bakterium zu bewirken, wobei der Operator gramnegativen bakteriellen Ursprungs ist; und

(b) eine DNA Sequenz, die einen gramnegativen bakteriellen Repressor des Operators von (a) unter der Steuerung eines bakteriellen Promotors kodiert;

das genannte Verfahren umfassend die Transformation des Bakteriums mit DNA, die die Elemente (a) und (b) enthält.

10. Verfahren nach Anspruch 9, worin die die Elemente (a) und (b) enthaltende, transformierende DNA auf das genannte Bakterium von einem unterschiedlichen bakteriellen Wirt übertragen wird.

11. Verfahren nach Anspruch 10, worin die transformierende DNA von einem gramnegativen bakteriellen Wirt übertragen wird.

12. Verfahren nach einem der Ansprüche 9 bis 11, worin der Operator der lac Operator ist und der Repressor lac I ist.

13. Verfahren nach einem der Ansprüche 9 bis 11, worin (a) die DNA Sequenz der B. licheniformis Penicillinase Promotor RNA Polymeraseerkennungstelle und den Operatorbereich des E. coli lac Promotor/Operators umfaßt.

14. Verfahren nach einem der Ansprüche 9 bis 11, worin der Promotor/Operator von (a) ein Hybrid ist, in dem der Promotor unter jenen ausgewählt ist, die dafür bekannt sind, wirksam im Wirtsorganismus zu sein, und der Operator ein induzierbarer Operator ist.

15. Verfahren nach Anspruch 14, worin (a) die DNA Sequenz der SPO-1 Phage Promotor RNA Polymeraseerkennungstelle und den Operatorbereich des E. coli lac Promotor/Operators umfaßt.

16. Verfahren nach einem der Ansprüche 9 bis 11, worin der Operator und Repressor ein E. coli Operator und Repressor ist.

# EP 0 130 074 B1

17. Verfahren nach einem der Ansprüche 9 bis 16, worin die Transformation mit einem Vektor durchgeführt wird, der sowohl das gegenständliche Kodierungssequenzen (a) als auch das Repressorgen (b) enthält.

18.Verfahren nach einem der Ansprüche 9 bis 17, worin das gegenständliche Kodierungssequenzgen (a) und das Repressorgan (b) in das Wirtsgenom einverleibt sind.

**Revendications**

1. Bactérie gram-positive transformée pour l'expression d'une séquence codante d'un sujet, la bactérie comprenant:

(a) un promoteur/opérateur bactérien lié de manière opérante àsune séquence codante d'un sujet, pour effectuer son expression dans la bactérie, l'opérateur étant d'origine bactérienne gram-négative; et

(b) une séquence d'ADN codant pour un répresseur bactérien gram-négatif de l'opérateur de (a) sous le contrôle d'un promoteur bactérien.

2. Bactérie transformée selon la revendication 1, où l'opérateur est l'opérateur lac et le répresseur est lac I.

3. Bactérie transformée selon la revendication 1, où (a) comprend la séquence d'ADN du site de reconnaissance de l'ARN polymérase du promoteur de pénicillinase de *B. Licheniformis* et la région opérateur du promoteur/opérateur lac de *E. coli*.

4. Bactérie transformée selon la revendication 1, où le promoteur/opérateur de la partie (a) est un hybride dans lequel le promoteur est sélectionné parmi ceux qui sont connus pour être efficaces dans l'organisme hôte et l'opérateur est un opérateur inductible.

5. Bactérie transformée selon la revendication 4, où (a) comprend la séquence d'ADN du site de reconnaissance de l'ARN polymérase du promoteur de phage SPO-1 et la région opérateur du promoteur/opérateur lac de *E. coli*.

6. Bactérie transformée selon la revendication 1, où l'opérateur et le répresseur sont un opérateur et un répresseur de *E. coli*.

7. Bactérie selon l'une quelconque des revendications précédentes, transformée avec un vecteur qui contient à la fois le gène (a) de séquence codant du sujet et le gène répresseur (b).

8. Bactérie transformére selon l'une quelconque des revendications précédentes, où le gène (a) de séquence codant du sujet et le gène répresseur (b) ont été incorporés dans le génome hôte.

9. Procédé pour la préparation d'une bactérie gram-positive transformée pour l'expression d'une séquence codant d'un sujet, la bactérie comprenant:

(a) un promoteur/opérateur bactérien lié de manière opérante à la séquence codant du sujet pour effectuer son expression dans la bactérie, l'opérateur étant d'origine bactérienne gram-négative; et

(b) une séquence d'ADN codant pour un répresseur bactérien gram-négatif de l'opérateur de (a) sous le contrôle d'un promoteur bactérien;

le procédé précité comprenant la transformation de la bactérie avec de l'ADN contenant les éléments (a) et (b).

10. Procédé selon la revendication 9, où l'ADN transformant contenant les éléments (a) et (b) est transféré dans la bactérie précitée à partir d'un hôte bactérien différent.

11. Procédé selon la revendication 10, où l'ADN transformant est transféré à partir d'un hôte bactérien gram-négatif.

12. Procédé selon l'une quelconque des revendications 8 à 11, où l'opérateur est l'opérateur lac et le répresseur est lac I.

13. Procédé selon l'une quelconque des revendications 9 à 11, où (a) comprend la séquence d'ADN du site de reconnaissance de l'ARN polymérase du promoteur de pénicillinase de *B. Licheniformis* et la région opérateur du promoteur/opérateur lac de *E. coli*.

14. Procédé selon l'une quelconque des revendications 9 à 11, où le promoteur/opérateur de la partie (a) est un hybride dans lequel le promoteur est sélectionné parmi ceux qui sont connus pour être efficaces dans l'organisme hôte et l'opérateur est un opérateur inductible.

15. Procédé selon la revendication 14, où (a) comprend la séquence d'ADN du site de reconnaissance de l'ARN polymérase du promoteur de phage SPO-1 et la région opérateur du promoteur/opérateur lac de *E. coli*.

16. Procédé selon l'une quelconque des revendications 9 à 11, où l'opérateur et le répresseur sont un opérateur et un répresseur de *E. coli*.

17. Procédé selon l'une quelconque des revendications 9 à 16, où la transformation est réalisée avec un vecteur qui contient à la fois le gène (a) de la séquence codant du sujet et le gène répresseur (b).

18. Procédé selon l'une quelconque des revendications 9 à 17, où le gène (a) de la séquence codant du sujet et le gène répresseur (b) sont incorporés dans le génome hôte.

Fig.1.

Fig.2.

Fig. 3.

BSA42 subclone

BamHI, PstI
650bp fragment
denature

Fig. 4.

## *Fig. 5.*

pBS42

−IPTG

+IPTG

Fig. 6.

## Fig.7.

pSPIF-Ⅱ →  BamHI, Pol I, EcoRI

BamHI, Pol I
ClaI, Pol I
Ppcn
lac I
LeIF-A
Pspac-I
pLIQ-I
EcoRI
EcoRI

pAIQ25 →  BamHI, Pol I, EcoRI

ClaI
Ppcn
lac I
BamHI, Pol I
XbaI, Pol I
pcn
Ppac-I
pAIQ120
(BamHI)
EcoRI
EcoRI

ClaI, Pol I / EcoRI

XbaI, Pol I / EcoRI

EcoRI  XbaI
BamHI
Sau3A
Pvu II
pBR322 origin
Sau3A
BamHI
pBS7
(BamHI)
neoʳ
ori
pUB110 vector

7

Pac-I

# Fig.8.

EcoRI
GAATTCGGTG GAAACGAGGT CATCATTTCC TTCCGAAAAA ACGGTTGCAT TTAAATCTTA CATATGTAAT

 $\quad$ <<<< << lac Op >>>>>> $\quad$ Sau3A $\qquad$ ***** $\qquad$ met
ACTTTCAATT GTGAGCGGAT AACAATTCCG GATCAATCAA ATATTCAAAC GGAGGGAGAC GATTTTGATG
‾‾‾  ‾‾‾‾‾‾‾‾‾ ‾‾‾‾‾‾‾‾‾

EcoRI
GAATTCTACA CAGCCCAGTC CAGACTATTC GGCACTGAAA TTATGGGTGA AGTGGTCAAG ACCTCACTAG

GCACCTTAAA AATAGCGCAC CCTGAAGAAG ATTTATTTGA GGTAGCCCTT GCCTACCTAG CTTCCAAGAA

AGATATCCTA ACAGCACAAG AGCGGAAAGA TGTTTTGTTC TACATCCAGA ACAACCTCTG CTAAAATTCC

 $\qquad$ <<< <<< lac Op
TGAAAAATTT TGCAAAAAGT TGTTGACTTT ATCTACAAGG TGTGGCATAA TGTGTGGAAT TGTGAGCGGA
‾‾‾‾‾‾‾‾‾ ‾‾‾‾‾‾‾‾‾

 >>>>>>HindIII **** * $\quad$ XbaI EcoRI met
TAACAATTAA GCTTAAGGAG GTGTATCTAG AATTCATG
‾‾‾‾‾‾‾‾ ‾‾‾‾‾‾‾‾‾ ‾‾‾‾‾‾‾‾‾ ‾‾‾‾‾‾‾‾

Spac-I

Fig.9.

Fig.10.

EP 0 130 074 B1

Fig.11.

# Fig.12.

GTGACCCACACC
GGTGTGG

GGGAAGCTCACCTGGGTGCCCAACGGTGCACCGGTTTCTGCACTTGACAACACAACCAAC
CCCTTCGAGTGGACCCACGGGTTGCCACGTGGCCAAAGACGTGAACTGTTGTGTTGGTTG


CCCACTGCATACCACAAGAGACCGCTGACTCGACTGGCTCTCCCATACACCGCGCCACAC
GGGTGACGTATGGTGTTCTCTGGCGACTGAGCTGACCGAGAGGGTATGTGGCGCGGTGTG


CGCGTGTTGGCCACGACGTACACTGGTACAACAACCTACACTACCAGTGCACGTAGAGGA
GCGCACAACCGGTGCTGCATGTGACCATGTTGTTGGATGTGATGGTCACGTGCATCTCCT


GATTTGGTCCATTTGGCGGCAGCACACGCTCGGCACTTGCCGACGTCGTTCAACTTTGGT
CTAAACCAGGTAAACCGCCGTCGTGTGCGAGCCGTGAACGGCTGCAGCAAGTTGAAACCA


GCAGTTAAAGCAGAAAAAGTCACTGAGCTGCTGGTGCGCATGAAGCGTGCAGAACTCTAT
CGTCAATTTCGTCTTTTTCAGTGACTCGACGACCACGCGTACTTCGCACGTCTTGAGATA


TGCCCCAGGCCGATTCCTCCGATTCGGCCAACGGGCGACAGACACAAGCAATCGTTTATC
ACGGGGTCCGGCTAAGGAGGCTAAGCCGGTTGCCCGCTGTCTGTGTTCGTTAGCAAATAG


G
CTTAA